(19) Europäisches Patentamt
European Patent Office
Office européen des brevets

(11) Veröffentlichungsnummer: **0 139 135 B1**

# (12) EUROPÄISCHE PATENTSCHRIFT

(45) Veröffentlichungstag der Patentschrift: **08.07.87**

(21) Anmeldenummer: **84109603.5**

(22) Anmeldetag: **13.08.84**

**ERRATUM**

| DIE TEXTSTELLE: / TEXT PUBLISHED: / LE PASSAGE SUIVANT: | (SEITE, SPALTE, ZEILE) / (PAGE, COLUMN, LINE) / (PAGE, COLONNE, LIGNE) | | LAUTET BERICHTIGT: / SHOULD READ: / DEVRAIT ETRE LU: |
|---|---|---|---|
| $R^3$—N=C=O | 2 | 55 | $R^6$—N=C=O |
| worin $R^3$ die | 2 | 57 | worin $R^6$ die |
| 1-N-Diethylcarbamoyl-4- | 3 | 40 | 1-N,N-Diethylcarbamoyl-4- |
| 4-Methyl-5-chlorimidazol und 47 | 3 | 60 | 4-Methyl-5-chlorimidazol 47 |
| $R^3$—N=C=O | 5 | 38 | $R^6$—N=C=O |
| worin $R^3$ die | 5 | 40 | worin $R^6$ die |

Tag der Entscheidung über die Berichtigung / Date of decision on rectification: / Date de décision portant sur modification: 19.9.88

Ausgabe- und Veröffentlichungstag: / Issue and publication date: / Date d'edition et de publication: 9.11.88

Patbl.Nr / EPB no: / Bull. no: 88/45

(19) Europäisches Patentamt
European Patent Office
Office européen des brevets

(11) Veröffentlichungsnummer : **0 139 135**
**B1**

(12) # EUROPÄISCHE PATENTSCHRIFT

(45) Veröffentlichungstag der Patentschrift : **08.07.87**

(51) Int. Cl.[4] : **C 07 D233/68**, C 09 K 17/00

(21) Anmeldenummer : **84109603.5**

(22) Anmeldetag : **13.08.84**

(54) **4-Alkylimidazol-Derivate, ihre Herstellung und Verwendung.**

(30) Priorität : **20.08.83 DE 3330192**

(43) Veröffentlichungstag der Anmeldung : **02.05.85 Patentblatt 85/18**

(45) Bekanntmachung des Hinweises auf die Patenterteilung : **08.07.87 Patentblatt 87/28**

(84) Benannte Vertragsstaaten : **AT BE CH DE FR GB IT LI NL SE**

(56) Entgegenhaltungen : **US-A- 4 233 058**

(73) Patentinhaber : **BASF Aktiengesellschaft**
**Carl-Bosch-Strasse 38**
**D-6700 Ludwigshafen (DE)**

(72) Erfinder : **Dockner, Toni, Dr.**
**Grossgasse 6**
**D-6701 Meckenheim (DE)**
Erfinder : **Pommer, Ernst-Heinrich, Dr.**
**Berliner Platz 7**
**D-6703 Limburgerhof (DE)**
Erfinder : **Dressel, Juergen, Dr. Dipl.-Landwirt**
**Medenheimer Strasse 18**
**D-6708 Neuhofen (DE)**

**EP 0 139 135 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 4-Alkylimidazol-Derivate, ihre Herstellung und ihre Verwendung als Nitrifikationshemmer.

Ammoniumstickstoff wird im Boden durch Bakterien aus den Gattungen Nitrosomonas und Nitrobacter über Nitritstickstoff zu Nitratstickstoff oxidiert. Das Ausmaß der Nitrifikation ist abhängig von der Bodenart, dem pH-Wert des Bodens, der Bodenfeuchtigkeit und der biologischen Aktivität des Bodens. Da der Nitratstickstoff im Gegensatz zum Ammoniumstickstoff vor allem auch auf leichteren Böden der Auswaschung unterliegt und damit für die Pflanzenernährung nicht mehr verfügbar ist und die Gefahr einer Nitrat-Anreicherung im Grundwasser besteht, kommt der Nitrifikationshemmung besondere Bedeutung zu.

Für diesen Zweck verwendete Handelsprodukte sind z. B. Dazomet (3,5-Dimethyltetrahydro-1,3,5-thiadiazin-2-thion) und Nitrapyrin (2-Chlor-6-trichlormethylpyridin ; vgl. Down to Earth 32, 14-17 (1976)) sowie Dicyandiamid (Landw. Forschung 27, 74-82 (1972)). Als Nitrifikationshemmer sollen auch gewisse Pyrimidin- und Pyrazolderivate wirken (vgl. DE-OS 2 745 833). Die bekannten Wirkstoffe genügen jedoch nicht allen Ansprüchen hinsichtlich ihrer Wirksamkeit, Selektivität und Wirkungsdauer, Wirtschaftlichkeit, Unschädlichkeit oder ihrer anwendungstechnischen Eigenschaften wie Wasserlöslichkeit, Dispergierbarkeit, Dampfdruck usw.

Es wurde nun gefunden, daß 4-Alkylimidazol-Derivate der Formel I

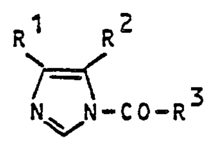

(I)

worin

$R^1$ eine $C_1$-$C_4$-Alkylgruppe,

$R^2$ ein Chlor- oder Bromatom und

$R^3$ eine —$OR^4$— oder —$NR^5R^6$-Gruppe bedeutet, wobei $R^4$ eine $C_1$-$C_4$-Alkylgruppe, $R^5$ ein Wasserstoffatom oder eine $C_1$-$C_4$-Alkylgruppe und $R^6$ eine $C_1$-$C_4$-Alkylgruppe oder einen gegebenenfalls durch bis zu 2 Halogenatome substituierten Phenylrest darstellen, sowie deren Salze gute Eigenschaften als Nitrifikationshemmer besitzen.

In der Formel I bedeutet $R^1$ vorzugsweise eine Methylgruppe, $R^2$ vorzugsweise ein Chloratom und $R^3$ vorzugsweise die Gruppen $NR^5R^6$, wobei R5 vorzugsweise ein Wasserstoffatom und $R^6$ vorzugsweise eine Methylgruppe oder einen gegebenenfalls durch bis zu 2 Chloratome substituierten Phenylrest darstellen.

Die neuen 4-Alkylimidazol-Derivate können erhalten werden, indem man

a) — falls $R^3$ eine $OR^4$— oder $NR^5R^6$-Gruppe mit $R^5$ in der Bedeutung einer $C_1$-$C_4$-Alkylgruppe darstellt — eine Verbindung der Formel II

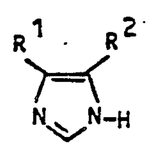

(II)

worin $R^1$ und $R^2$ die angegebene Bedeutung besitzen, mit einer Verbindung der Formel III

$$X—CO—R^3 \qquad (III)$$

worin $R^3$ die oben angegebene Bedeutung hat und X ein Halogenatom ist, in Gegenwart einer Base umsetzt oder

b) — falls $R^3$ eine $NHR^6$-Gruppe darstellt — eine Verbindung der Formel II mit einem Isocyanat der Formel IV

$$R^3—N=C=O \qquad (IV)$$

worin $R^3$ die angegebene Bedeutung hat, umsetzt.

Aus der Formel I ergeben sich für den Fachmann ohne weiteres noch andere Herstellmöglichkeiten, auf die hier nicht eingegangen wird.

Die Umsetzung a) wird in der Regel in Gegenwart einer äquivalenten Menge an Base durchgeführt. Als Basen eignen sich insbesondere tertiäre Amine — wie Trimethylamin oder Triethylamin — sowie Alkali- oder Erdalkalihydroxide bzw. -alkoholate. Als Lösungsmittel eignen sich z. B. Chloroform, Toluol,

Xylol, Chlorbenzole. Die Reaktion gelingt in der Regel bei einer Temperatur von 40 bis 100 °C. Zweckmäßigerweise führt man sie bei der Rückflußtemperatur des verwendeten Lösungsmittels durch.

Die Umsetzung b) wird in einem unter den Reaktionsbedingungen inerten Lösungsmittel durchgeführt. Als solche eignen sich insbesondere Kohlenwasserstoffe und Halogenkohlenwasserstoffe im Siedebereich von 40 bis 140 °C, vorzugsweise 60 bis 110 °C, sowie Acetonitril und Dimethylformamid. Die Umsetzung findet in der Regel unter Rückflußbedingungen statt.

Die für die Reaktionen benötigten Ausgangsstoffe der Formel II sind durch Umsetzung der entsprechenden Imidazole mit Halogen oder Hypohalogenit zugänglich (vgl. DE-OS 31 45 927).

Die erfindungsgemäßen Substanzen können als solche, d. h. als freie (sehr schwache) Basen oder als Salz einer biologisch annehmbaren Säure, vorzugsweise als Phosphat, Sulfat, Acetat, Citrat, Tartrat, insbesondere als Hydrochlorid, allein oder im Gemisch mit festen oder flüssigen Düngemitteln, die Ammoniumstickstoff, Harnstoff oder Ammoniak enthalten, angewendet werden ; auch ist die Ausbringung gemeinsam mit Pflanzenbehandlungs-oder Bodenverbesserungsmitteln möglich. Zweckmäßigerweise werden die Wirkstoffe gleichzeitig mit dem Düngemittel ausgebracht. Die Aufwandmengen betragen 0,05 bis 10 kg/ha, vorzugsweise 0,5 bis 3 kg/ha. Bei der mit festen oder flüssigen Düngemitteln kombinierten Anwendung können die Wirkstoffe in Mengen von 0,5 bis 10 Gewichtsprozent, bezogen auf Düngemittelstickstoff, eingesetzt werden.

Die erfindungsgemäßen Nitrifikationshemmer sind sehr wirksam, ungiftig, nicht flüchtig, ausreichend wasserlöslich und stabil. Sie verbleiben lange im Boden, bleiben also lange wirksam. Sie dienen somit nicht nur dem Umweltschutz, indem sie das Auswaschen von Nitrat in das Grundwasser verhindern, sondern auch einer wesentlichen Verbesserung der Düngerverwertung, insbesondere bei leichteren Böden.

### Beispiel 1

In einem Rührkolben mit Thermometer und Rückflußkühler wurden zu 233 g 4-Methyl-5-chlorimidazol in 1 Liter Chloroform 114 g Methylisocyanat zugetropft. Die Temperatur stieg dabei bis 40 °C an. Man kochte 2 h unter Rückfluß und dampfte die Reaktionslösung im Vakuum ein. Der Rückstand wurde in 400 ml Toluol heiß gelöst, mit Kohle filtriert und gekühlt. Die ausgeschiedenen Kristalle wurden abgesaugt, mit Toluol gewaschen und getrocknet. Man erhielt 237 g (68,3 %) 1-N-Methylcarbamoyl-4-methyl-5-chlorimidazol, Fp. 94,1 bis 94,4 °C.

### Beispiel 2

In einem Rührkolben mit Thermometer und Rückflußkühler wurden zu 58,5 g 4-Methyl-5-chlorimidazol in 200 ml Chloroform, 76 g Triethylamin und danach 68 g Diethylcarbaminsäurechlorid zugetropft. Anschließend wurde 2 h unter Rückfluß gekocht, abgekühlt und das ausgefallene Triethylamin-hydrochlorid abgesaugt. Das Filtrat wurde unter vermindertem Druck eingedampft und der Rückstand mit 500 ml Aceton versetzt. Unter Eiskühlung wurde HCl-Gas eingeleitet. Es entstand ein Niederschlag, der abgesaugt und getrocknet wurde. Man erhielt 66 g (52,4 %) 1-N-Diethylcarbamoyl-4-methyl-5-chlorimidazol-hydrochlorid, Fp. ab 208 °C unter Zersetzung.

### Beispiel 3

Analog Beispiel 1 wurden zu 58,5 g 4-Methyl-5-chlorimidazol in 250 ml Chloroform 59,5 g Phenylisocyanat getropft. Die Temperatur stieg dabei bis 42 °C an. Anschließend kochte man 2 h unter Rückfluß und kühlte dann mit Eis ab. Der ausgefallene Niederschlag wurde abgesaugt und mit Chloroform nachgewaschen. Man erhielt nach dem Trocknen 80 g (67,9 %) 1-N-Phenylcarbamoyl-4-methyl-5-chlorimidazol, Fp. 112,3 bis 114,4 °C.

### Beispiel 4

Analog Beispiel 1 wurden zu 29 g 4-Methyl-5-chlorimidazol in 150 g Chloroform 47 g 3,4-Dichlorphenylisocyanat, welches in 50 g Chloroform gelöst war, getropft. Man kochte 2 h unter Rückfluß, kühlte ab und saugte den erhaltenen Niederschlag ab. Nach dem Trocknen erhielt man 60 g (78,9 %) 1-N-(3,4-Dichlorphenyl)-carbamoyl-4-methyl-5-chlorimidazol, Fp. 143,7 bis 144,7 °C.

### Beispiel 5

Analog Beispiel 1 wurden zu 29 g 4-Methyl-5-chlorimidazol und 47 g 3,5-Dichlorphenylisocyanat, in je 100 g Chloroform gelöst, getropft. Dann kochte man 2 h unter Rückfluß, kühlte ab und saugte den Rückstand ab. Man wusch mit Chloroform nach, trocknete das Produkt und erhielt 60 g (78,8 %) 1-N-(3,5-Dichlorphenyl-carbamoyl)-4-methyl-5-chlorimidazol, Fp. 136,9 bis 138,6 °C.

### Beispiel 6

Analog Beispiel 1 wurden zu 40 g 4-Methyl-5-bromimidazol in 200 g Chloroform 13 g Methylisocyanat getropft. Die Temperatur stieg dabei bis 35 °C an. Nachdem man 2 h unter Rückfluß gekocht hatte, wurde die Reaktionslösung unter vermindertem Druck eingedampft. Man erhielt 55 g Rückstand, den man aus 110 ml Toluol umkristallisierte. Nach dem Abkühlen und Absaugen des Produktes wurde getrocknet. Man erhielt 30 g (55 %) 1-[N-Methyl-carbamoyl]-4-methyl-5-bromimidazol, Fp. 98,4 bis 100,1 °C.

Beispiel 7

Analog Beispiel 1 wurden 582,5 g 4-Methyl-5-chlorimidazol in 1 500 ml Toluol bei 70 °C gelöst. In diese Lösung ließ man 313,5 g Methylisocyanat langsam laufen. Dabei stieg die Temperatur bis etwa 90 °C an. Anschließend kochte man 30 min unter Rückfluß und kühlte dann ab. Unter Eiskühlung wurde 1 h nachgerührt und der ausgefallene Niederschlag abgesaugt. Man wusch mit Ether nach, trocknete und erhielt 826 g (95,2 %) 1-N-Methylcarbamoyl-4-methyl-5-chlorimidazol, Fp. 95,6 °C.

Beispiel 8

58,5 g 4-Methyl-5-chlorimidazol wurden in einer wie in Beispiel 1 beschriebenen Rührapparatur in 200 g Chloroform gelöst. 76 g Triethylamin wurden zugegeben und über einen Tropftrichter 54,5 g Chlorameisensäureethylester zugetropft. Die Temperatur erhöhte sich dabei bis zum Siedepunkt.

Anschließend kochte man 2 Stunden unter Rückfluß, kühlte auf 0 °C ab und saugte das ausgefallene Triethylamin-Hydrochlorid ab (53 g).

Das Filtrat wurde unter vermindertem Druck eingedampft, und man erhielt als Rückstand ein Öl, das zum Teil kristallisierte. Dieser Rückstand wurde in Aceton digeriert, abgesaugt und getrocknet. So wurden weitere 15 g Triethylamin-Hydrochlorid erhalten. Das Filtrat wurde wieder unter vermindertem Druck eingedampft, und aus dem Rückstand erhielt man nach Destillation 64 Teile 1-Ethoxicarbonyl-4-methyl-5-chlorimidazol (Kp bei 40 Pa 92 °C), was einer Ausbeute von 68 % d. Th. entspricht.

Anwendungsbeispiel

Zu 200 g eines lehmigen Sandbodens, dessen Feuchtigkeitsgehalt auf 50 % der maximalen Wasserkapazität eingestellt war, wurden 220 mg Ammoniumsulfat gegeben und gründlich vermischt. Danach wurden die Wirkstoffe, in 0,2 ml Aceton gelöst, in Mengen von 1 ppm, bezogen auf feuchten Sandboden, zugesetzt. Nach sorgfältiger Durchmischung wurden die Bodenproben nach Verdunsten des Acetons in mit Aluminiumfolie zur Vermeidung von Wasserverlusten abgedeckten 1 1-Gläsern zusammen mit den Kontrollen ohne Wirkstoffzusatz über einen Zeitraum von 28 Tagen bei 21 °C bebrütet.

Danach wurden jeweils 2,5 g der Bodenproben in 100 ml-Erlenmeyerkolben eingefüllt und 22,5 ml einer 0,1 n Kaliumsulfat-Lösung zugesetzt. Nach 30-minütigem Schütteln wurde abfiltriert und jeweils 2,5 ml der Bodenauszüge wurden mit 1 625 ml aqua dest. vermischt. Anschließend wurden zum Nachweis der im Bodenauszug noch vorhandenen Ammoniumionen 1,25 ml Neßler-Reagenz hinzugefügt und gründlich geschüttelt. Die Farbveränderungen wurden dann photometrisch bei einer Wellenlänge von 420 nm gemessen. Anhand von Standardkurven, die durch Messung von Lösungen mit bekannten Ammoniumsulfat-Gehalten ermittelt worden waren, wurden die noch in den Bodenproben vorhandenen Ammoniumsulfat-Mengen bestimmt. Die prozentuale Hemmung der Nitrifikation in den behandelten Bodenproben wurde im Vergleich mit den unbehandelten Bodenproben (nur Ammoniumsulfat-Zusatz) nach folgender Formel berechnet :

$$\ldots \% \text{ Hemmung der Nitrifikation} = (a - b)/a \cdot 100$$

a = Nitrifikationsrate von Ammoniumsulfat
b = Nitrifikationsrate von Ammoniumsulfat + Nitrifikationshemmer

Die folgende Tabelle zeigt die erhaltenen Ergebnisse. Als Vergleichssubstanz (A) diente die in der DE-OS 2 745 833 beschriebene Substanz Nr. 95 (1-Phenoxycarbamoyl-3-methylpyrazol).

| Wirkstoff Beispiel | ... % Hemmung der Nitrifikation 4 Wochen nach Zugabe von 1 ppm Wirkstoff zum Boden |
|---|---|
| 1 | 94 |
| 3 | 85 |
| 4 | 71 |
| A | 69 |

**Patentansprüche**

1. 4-Alkylimidazol-Derivate der Formel I

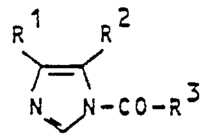

(I)

worin

$R^1$ eine $C_1$-$C_4$-Alkylgruppe,

$R^2$ ein Chlor- oder Bromatom und

$R^3$ eine —$OR^4$— oder —$NR^5R^6$-Gruppe bedeutet, wobei $R^4$ eine $C_1$-$C_4$-Alkylgruppe, $R^5$ ein Wasserstoffatom oder eine $C_1$-$C_4$-Alkylgruppe und $R^6$ eine $C_1$-$C_4$-Alkylgruppe oder einen gegebenenfalls durch bis zu 2 Halogenatome substituierten Phenylrest darstellen, und deren Salze.

2. Verfahren zur Herstellung der Verbindungen der Formel I gemäß Anspruch 1, dadurch gekennzeichnet, daß man

a) — falls $R^3$ eine $OR^4$— oder $NR^5R^6$-Gruppe mit $R^5$ in der Bedeutung einer $C_1$-$C_4$-Alkylgruppe darstellt — eine Verbindung der Formel II

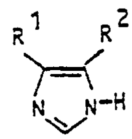

(II)

worin $R^1$ und $R^2$ die oben angegebene Bedeutung besitzen, mit einer Verbindung der Formel III

$$X—CO—R^3 \qquad (III)$$

worin $R^3$ die oben angegebene Bedeutung hat und X ein Halogenatom ist, in Gegenwart einer Base umsetzt oder

b) — falls $R^3$ eine $NHR^6$-Gruppe darstellt — eine Verbindung der Formel II mit einem Isocyanat der Formel IV

$$R^3—N=C=O \qquad (IV)$$

worin $R^3$ die oben angegebene Bedeutung hat, in Gegenwart eines Lösungsmittels umsetzt.

3. Verfahren zur Hemmung der Nitrifikation von Ammoniumstickstoff im Boden, dadurch gekennzeichnet, daß man 0,05 bis 10 kg/ha einer Verbindung der Formel I gemäß Anspruch 1 in den Boden einbringt.

4. Nitrifikationshemmer, enthaltend eine Verbindung der Formel I gemäß Anspruch 1.

**Claims**

1. A 4-alkylimidazole derivative of the formula I

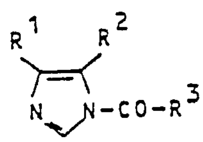

(I)

where

$R^1$ is $C_1$-$C_4$-alkyl,

$R^2$ is chlorine or bromine, and

$R^3$ is an —$OR^4$ or —$NR^5R^6$ group in which $R^4$ is $C_1$-$C_4$-alkyl, $R^5$ is hydrogen or $C_1$-$C_4$-alkyl, and $R^6$ is $C_1$-$C_4$-alkyl or is phenyl which is unsubstituted or substituted by 1 or 2 halogen atoms, and its salts.

2. A process for the preparation of a compound of the formula I as claimed in claim 1, wherein

a) when $R^3$ is an $OR^4$ or $NR^5R^6$ group in which $R^5$ is $C_1$-$C_4$-alkyl, a compound of the formula II

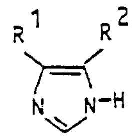

(II)

where $R^1$ and $R^2$ have the above meanings, is reacted with a compound of the formula III

$$X—CO—R^3 \quad (III)$$

where $R^3$ has the above meanings and X is halogen, in the presence of a base, or

b) when $R^3$ is an $NHR^6$ group, a compound of the formula II is reacted with an isocyanate of the formula IV

$$R^3—N=C=O \quad (IV)$$

where $R^3$ has the above meanings, in the presence of a solvent.

3. A process for inhibiting the nitrification of ammonium nitrogen in the soil, wherein from 0.05 to 10 kg/ha of a compound of the formula I as claimed in claim 1 is introduced into the soil.

4. A nitrification inhibitor containing a compound of the formula I as claimed in claim 1.

**Revendications**

1. Dérivés d'alkyl-4 imidazoles de la formule I

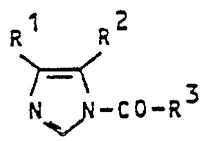

(I)

dans laquelle

$R^1$ désigne un radical alkyle en $C_1$ à $C_4$,

$R^2$ désigne un atome de chlore ou de brome et

$R^3$ désigne un groupe $—OR^4$ ou $—NR^5R^6$, où $R^4$ désigne un radical alkyle en $C_1$ à $C_4$, $R^5$ un atome d'hydrogène ou un radical alkyle en $C_1$ à $C_4$ et $R^6$ un radical alkyle en $C_1$ à $C_4$ ou un groupe phényle éventuellement mono- ou di-halo-substitué, ainsi que les sels de ces dérivés.

2. Procédé de préparation des composés de la formule I selon la revendication 1, caractérisé en ce que l'on fait réagir

a) lorsque $R^3$ représente un groupe $—OR^4$ ou $—NR^5R^6$ avec $R^5$ = radical alkyle en $C_1$ à $C_4$ : un composé de la formule II

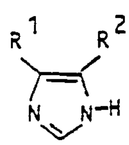

(II)

dans laquelle $R^1$ et $R^2$ possèdent la signification définie, en présence d'une base avec un composé de la formule III

$$X—CO—R^3 \quad (III)$$

dans laquelle $R^3$ possède la signification définie et X désigne un atome d'halogène ;

b) lorsque $R^3$ représente un groupe $—NHR^6$ : un composé de la formule II dans un solvant avec un isocyanate de la formule IV

$$R^3—N=C=O \quad (IV)$$

dans laquelle $R^3$ possède la signification définie.

3. Procédé pour inhiber la nitrification de l'azote ammoniacal dans le sol, caractérisé en ce que l'on incorpore au sol entre 0,05 et 10 kg d'un composé de la formule I selon la revendication 1 par hectare.

4. Inhibiteur de la nitrification, comprenant un composé de la formule I selon la revendication 1.